# EUROPEAN PATENT SPECIFICATION

(11) **EP 3 758 796 B1**
(45) Date of publication and mention of the grant of the patent: **11.10.2023**
(21) Application number: 19706463.7
(22) Date of filing: 14.02.2019
(51) Int. Cl.: A61N 1/375, A61N 1/372, A61B 17/34

(54) **RECAPTURE ASSEMBLY FOR A CATHETER SYSTEM AND CATHETER SYSTEM FOR RETRIEVAL OF AN IMPLANT**
ZURÜCKGEWINNUNGSANORDNUNG FÜR EIN KATHETERSYSTEM UND KATHETERSYSTEM ZUM ZURÜCKHOLEN EINES IMPLANTATS
ENSEMBLE DE RECAPTURE POUR UN SYSTÈME DE CATHÉTER ET SYSTÈME DE CATHÉTER DE RÉCUPÉRATION D'UN IMPLANT

(30) Priority: 02.03.2018 US 201862637435 P
(43) Date of publication of application: 06.01.2021
(73) Proprietor: BIOTRONIK SE & Co. KG, 12359 Berlin (DE)
(72) Inventor: TAFF, Brian M., Portland, Oregon 97217 (US); KIBLER, Andrew B., Lake Oswego, Oregon 97035 (US)
(74) Representative: Biotronik Corporate Services SE
(86) International application number: PCT/EP2019/053690
(87) International publication number: WO 2019/166230

(56) References cited:
- EP-A1- 3 125 995
- EP-B1- 3 125 995
- WO-A1-2017/058315
- US-A1- 2016 213 919
- US-A1- 2016 220 829
- US-A1- 2016 279 423

## Description

The invention relates to a recapture assembly for a catheter system and a respective catheter system for retrieval of an implant.

Minimally invasive procedures using catheter systems have been implemented in a variety of medical settings, e. g. for vascular interventions, such as angioplasty, stenting, embolic protection, electrical heart stimulation, heart mapping and visualization, and the like. These procedures generally rely on accurately navigating and placing instruments or implants within a patient's vasculature, for example by means of a catheter.

A prominent example of an implant is a cardiac pacemaker which uses electrical pulses, delivered by electrodes contracting the heart muscles, to regulate the beating of the heart. The primary purpose of a pacemaker is to maintain an adequate heart rate, either because the heart's natural pacemaker is not fast enough, or because there is a block in the heart's electrical conduction system. Modern pacemakers are externally programmable and allow a cardiologist to select the optimum pacing modes for individual patients. Some pacemakers combine a pacemaker and defibrillator in a single implantable device or stimulate different positions within the heart to improve synchronization of the lower chambers (ventricles) of the heart. The above mentioned artificial pacemaker is different from the heart's own natural pacemaker. The disclosure particularly refers to a cardiac pacemaker of leadless type, or other implants which need to be retrieved similarly to a leadless cardiac pacemaker. Self-contained or leadless pacemakers are typically fixed to an intracardial site by an actively anchoring mechanism such as a screw or a helical member that screws into the myocardium. Other anchoring means such as tine based anchors may be used as well. Leadless pacemakers do not need any leads as conventional pacemakers that conduct the electrical signals from a remote pulse generator to the intracardial site.

Leadless pacemakers or other implants are associated with two common surgical operations: implantation and recapture/explantation. To facilitate these operations with minimally invasive procedures, in case of a leadless pacemaker a long catheter is inserted via the femoral vein and guided to reach the inferior vena cava, where it is then steered into the right atrium and ventricle.

Some of the present catheter systems are instrumented such that they are capable of performing both the delivery and recapturing functions. Medtronic Micra Transcatheter Pacing System (TPS) and Micra Delivery Catheter provisions a means for enabling recapture as an extensible capability for its leadless implantation tool. The Medtronic solution, as described in their user manual, leverages an approach that permanently stations a rotatable, telescoping tube within the tooling. This steerable, telescoping tube resides within the catheter system at all times, serving responsibility for both implantation and recapture. As part of this telescoping tube, the Medtronic design presents an implant-facing terminus with a conically-shaped, ramping feature that transitions dimensionally from the outer diameter of the telescoping tube to an enlarged diameter compatible with the girth of the leadless pacer. The modular insert from Medtronic passes through the inner lumen of this permanently-stationed telescoping tube to introduce a snare and affiliated cinch tube to the implantation tool. This rotatable and telescoping snare/cinch combination expands the design's capabilities for reestablishing suture based tethered connections between the implant and the catheter-based tooling system. Following recapture with the snare/cinch combination, the tether linkage is shortened to force the implant to engage with the telescoping tube (the one always present within the tooling) such that the implant abuts with the tube's built-in non-deployable ramping feature.

Because the Medtronic design always retains a telescoping tube within the tooling, regardless of whether it is used for implantation or device recapture, the optimal ramping features at the tube's implant-facing terminus have centered on rigid, fixed, cup-based conical formats. Such formats arise largely due to the fact that there is no need (and no ability) to accommodate passing a large diameter structure through the narrow inner lumen of the catheter system. Such formatting unfortunately tends to enlarge the total length of rigid mated bodies that the system needs to route through the patient's vasculature from the IVC (inferior vena cava) and into the RV (right ventricle) during implantation, placing added pressure on efforts to shorten device length. This condition amounts to system design restrictions one must pay penalties for throughout the lifetime of the implanted condition (i.e. having a shorter than desired device with less primary cell capacity) despite the fact that such restrictions only emerge during the short-duration procedures necessary for device installation.

As explained above, to expand the catheter's functional capacity for recapture support, the Medtronic approach introduces the snare and the cinch tube within the inner lumen of the telescoping tube (again, the one always present with the ramping feature). This design choice means that three separate components, namely 1.) telescoping tube, 2.) the telescoping cinch tube, and 3.) the rotatable, telescoping snare are needed to enable recapture - a hardware stack-up in direct competition with efforts to minimize the catheter dimensions routed through the patient vasculature. In other words, the constant presence of the telescoping tube and its need to accommodate the passage of a snare/cinch assembly through its inner lumen fails to optimize the design of the catheter body around accessible, reduced cross-sectional profiles. Additionally, two of the three components used for recapture present articulations with largely overlapping responsibilities (i.e. telescopic movements) which again sidesteps potential paths toward optimized design. Last, because the entire recapture system lacks any additional built-in steering support beyond that offered by the implantation tool, reestablishing tether based linkages between the catheter and the implant 1.) relies upon the steering affordances native to the surrounding implantation catheter and 2.) assumes that patient vasculature and heart anatomy will consistently present conditions where the offset and distance between the implant-facing tip of the tooling and the leadless pacer is not excessive. Document WO-A-2017/058315 discloses an assembly according to the preamble of claim 1.

Document WO 2012/082755 A1 explains and shows a catheter system for retrieving a leadless pacemaker from a patient. The known catheter comprises a handle, a catheter shaft coupled to the handle, a snare disposed within the catheter shaft and extendable distally beyond the catheter shaft, a docking cap disposed on a distal portion of the catheter shaft, the docking cap being rotatable independently of the catheter shaft, and a torque shaft disposed within the catheter shaft, wherein the torque shaft is selectively connectable to the docking cap. The torque shaft is configured to rotate within the catheter shaft to apply rotational torque to the docking cap when connected to the docking cap. The snare is configured to grasp the cardiac leadless pacemaker. In embodiments where the snare includes a plurality of loops, it may be more likely that one of the loops will grasp the pacemaker than in embodiments where the snare comprises only a single loop. The docking cap is configured to allow docking of the leadless pacemaker with the retrieval catheter after engaging the pacemaker with the snare. The document further discloses that in some embodiments, the snare locking sleeve comprises a torque shaft that runs through the length of the catheter. This torque shaft can be rotated independently of the catheter shaft and coupled to the docking cap to apply rotational torque to the docking cap and, thus, to a pacemaker or medical device to be retrieved. This device exhibits the same problems as the Medtronic device discussed above. Additionally, in some cases it might be difficult to position the leadless cardiac pacemaker inside the docking cap because the proximal end of the leadless cardiac pacemaker may be seized behind the distal rim of the docking cap when it moves into the direction of the docking cap and is pulled by the snare into proximal direction.

Therefore it is an object of the invention to provide a catheter system which is safer in operation and makes the recapture of an implant straightforward and user-friendly while at the same time minimizing the length of inflexible components at the distal end of the tooling.

The above problem is solved by a recapture assembly for a catheter system comprising:
- a cinch tube with a centering device provided at the distal end of the cinch tube and
- a tether located within the cinch tube and adapted to be telescoped from the distal end of the cinch tube.

In one embodiment, the cinch tube is configured to be steerable by a steering element, for example by a pull wire, wherein the pull wire is accessible in the handle of the catheter system, wherein the steering element routes within the wall of the cinch tube.

With regard to the present invention the distal direction is the direction pointing away from the healthcare professional (HCP) conducting explantation (or the implantation) of an implant, e.g. a leadless cardiac pacemaker. The proximal direction is the opposite direction of the distal direction.

The recapture assembly serves to expand the baseline functionality of the catheter system to access essential device recapture dynamics. The recapture assembly eliminates - compared against the known approach - the need for a permanently-stationed telescoping tube within the catheter system.

The inventors recognized that a catheter system instrumented such that it is capable of performing both the delivery and recapture functions provides less degree of freedom to permit recapture barring the incorporation of unwanted complexity within the handle design used for delivery. They have further realized that a full independent retrieval catheter is possible but expensive and complicated. They found an solution which provides a recapture assembly which may be added to the delivery catheter system as a modular component only when retrieval is necessary. Preferably, the recapture assembly is insertable into the delivery catheter system such that the catheter remains in the vein, if it is already in place during an operation. The recapture assembly usually is attached to and within an outer sheath, e.g. an outer catheter shaft, of a catheter system such that it can be moved out of the distal end of the catheter shaft. The solution has the advantage that for both delivery and retrieval operation, the length of the inflexible distal portion of the catheter tooling is as short as possible to allow it to be steered and turned from the inferior vena cava around through the atrium and down into the ventricle of the heart. The present invention eliminates the demand for an implantation tooling to support device recapture needs until such needs are specifically called upon within the clinical setting. In turn, when recapture needs arise, the present invention ensures that the tooling successfully renders an expanded set of articulations and hardware features that make such procedures straightforward and user-friendly. By enabling these expanded functionalities, the approach avoids any need for the implantation tooling to incorporate hand controls and/or articulations specific to recapture duties as part of its baseline tooling design. Furthermore, by corralling such recapture support into a separate dedicated module, i.e. the recapture assembly, the complexity of the recapture articulations can be expanded beyond those that would be sensible in an all-in-one tooling approach. With this type of functional support in play, the steerable, telescopic recapture assembly can readily establish tethered connections between the tooling and the implant even when gross misalignments between the two pieces of hardware arise - subject even to the best case baseline implantation catheter positioning efforts.

The centering device, which may be configured deployable, is accommodated at the distal end of the cinch tube facilitates the introduction of the recapture assembly through the narrow inner lumen of the catheter system with an outer catheter shaft with the luxury that, in an embodiment, it can be subsequently dimensionally enlarged (i.e. activated) following its exodus from the implant-facing end (i.e. the distal end) of the catheter system or moving into an enlarged section of the outer catheter shaft. The centering device, in turn, forces recaptured implants to axially align with the catheter shaft in ways that soften the approach of the two pieces of hardware as the tether connection is retracted. Such a capability overcomes a binding response that commonly confounds complete functional reengagement between implants and their affiliated tooling catheters. All these capabilities in combination facilitate recapture and tooling engagement responses that ultimately support the safe and reliable retrieval of implants such as leadless cardiac pacemakers from patient vasculature. The centering device enables improved alignment between the implant body and the recapture assembly as necessary for safe implant recovery during explantation procedure.

The cinch tube enables closure of the tether in a fashion akin to that of the knot in a necktie. It may be highly flexible to enter/access the full extents of the RV and via built-in steering support better bridge the gap between the recapture assembly and the implant itself.

Accordingly, in an embodiment the centering device is adapted to be activated by moving the steerable cinch tube and/or the tether, for example by moving a distal end of the cinch tube relative the outer catheter shaft into distal direction beyond a distal end of the outer catheter shaft or into a section of the outer catheter shaft with a greater diameter. Activation of the centering device changes the centering device from a retracted state in which it has a reduced diameter that facilitates passing through the inner lumen of the catheter system into a deployed state for centering the implant. In another embodiment the centering device may be activated by moving (pulling) a wire provided at the cinch tube, for example for steering, or the tether into proximal direction relative to the outer catheter shaft, for example after the cinch tube was moved into distal direction and protrudes from a distal end of an outer catheter shaft. The user may use other pulling elements but in one embodiment pull wire accessible in the handle of the catheter system, wherein the pulling element routes within the wall of the cinch tube connected to the centering device for activation, for example by pulling the pull wire.

The tether forms at least one loop large enough to encircle the full implant and when closed via cinch tube it is enabled to directly engage with the implant's hitch.
the centering device comprises a plurality of wires (arms, struts), wherein each wire is attached with its first end to the distal end of the cinch tube or a fixed ring accommodated at the outer surface of the cinch tube and with its second end to a slidable ring accommodated at the outer surface of the cinch tube, wherein a first, most proximal position of the slidable ring each wire lies at least partially against the outer surface of the cinch tube and in a second, more distal position of the slidable ring compared with the first position each wire is folded forming at least one knee that protrudes (projects) outwards from the cinch tube. In the first position the wires may be biased by the outer catheter shaft, wherein in the second position the wires are released so that they are automatically folded, for example by moving the cinch tube out of the distal end of the outer catheter shaft, thereby causing the sliding movement of the ring. Alternatively, the cinch tube or the slidable ring may be actively moved in order to fold the wires. This embodiment of a centering device is little more complex and cost intensive than the above described embodiment but provides a structure producing higher radial force for implant alignment into a centered position with regard to a shaft of the catheter system accommodating the recapture assembly than the above mentioned embodiment comprising the plurality of radial arms. Hence, this embodiment works more reliable.

The radial arm, ring or wire may be formed by a polymer arm, wire or ring, stiff whisker-like arm, ring or wire or a Nitinol arm, ring or wire.

In one embodiment, the recapture device may comprise an additional (non-steerable) tube which enables snare cinching independent of the cinching enabled by the above-described first steerable tube. The additional (non-steerable) tube may be accommodated within the first cinch tube and form together with the cinch tube and the tether the recapture assembly. The tether is located within the additional non-steerable tube.

The centering devices described above may be manufactured using a common material and dedicated molding process. The sprocket and strut configurations (radial arm/wire) may be build using compliant but preformed polymers or metals. In the case of metal based implementations, either stainless steel or nitinol-based assemblies would adequately service the intended functionality.

The above task is further solved by a catheter system comprising at least one outer sheath, for example an outer shaft, and the recapture assembly as described above as a modular insert adapted to be passed through the proximal (clinician facing) terminus of the catheter system and the outer sheath, preferably during leaving the catheter system in the patient's body. The catheter system may comprise one or all of the above advantages. The recapture assembly is fed through the inner lumen of the tooling but springs into a more expanded condition by activating the centering device following its exodus from the catheter. Other embodiments may involve a tightening process to motivate the dimensional expansion of the centering device. More complex strategies may utilize a dedicated actuation via a posturing or even deployment on the basis of temperature changes affiliated with shape memory alloys.

In an embodiment the recapture assembly is adapted to telescope from the distal end of the outer sheath. In a further embodiment, an inner diameter of a distal section of the outer sheath is greater than an inner diameter of another section that follows proximal from this distal section of the outer sheath. The distal section of the outer sheath may be called implant protector cup as the implant may be accommodated within this section during explantation and withdrawal. Additionally, the enlarged distal section of the outer sheath (e.g. an outer shaft) may serve to activate the centering device of the recapture assembly as described above.

The above task is further solved by a method for retrieval of an implant comprising a hitch-like element at the implant's proximal end by the above described catheter system, wherein the method comprises the following steps:
- pairing (installing , e.g. attaching) the recapture assembly with the catheter system, for example while leaving the catheter system in the patient's body,
- if applicable, introducing the catheter system into the patient's body and moving the distal end of the catheter system within the vasculature of the patient until it is in close proximity to the implant to be explanted,
- telescoping the tether and the cinch tube, which may be configured steerable, from the distal end of the outer sheath, telescoping the tether from the distal end of the cinch tube and capture the hitch-like element at the implant, for example by lying the tether around the hitch-like element, and retracting the tether with the implant into proximal direction, activating the centering device of the cinch tube, whereby the centering device of the cinch tube is activated during at least one of the steps of this bullet point, and
- retracting the cinch tube into or within the outer sheath of the catheter system.

The recapture assembly provides a steering capability within the tether that is an extension of that offered by the baseline implantation catheter.

In one embodiment during the last step mentioned above during retracting the cinch tube, also the tether and the implant may be retracted into the outer sheath of the catheter system.

In one embodiment of the above method, the centering device of the cinch tube is activated by telescoping the cinch tube with regard to the outer sheath of the catheter assembly such that the cinch tube projects from the distal end of the outer sheath thereby transferring the plurality of radial arms from the first state into the second state or deflecting the plurality of wires radially outwards thereby forming a knee that protrudes radially outwards from the cinch tube. As indicated above, the biased radial arms are deflected when they move out of the distal end of the outer sheath or into a distal section of the outer sheath with an enlarged diameter.

The radial arms or wires may change their state from the first position into the second position using a shape-memory configuration of the arms or wires used to make the centering device. Once the centering feature was pushed out of a portion of the catheter where the surrounding outer diameter was larger than feasible to compress the arms or wires, the centering feature would auto-deploy.

Alternatively, the centering device is activated by movement of the slidable ring from the first into the second position which may be realized using a pull-wire connected with the slidable ring within the recapture assembly adapted to be actuated via user interaction at the handle that forced the slidable ring toward the fixed ring. Such an actuation could either embody a "one and done" irreversible operation or a response that was reversible via a built in strut shape configuration. The slidable ring may be moved as indicated above and/or facilitate stabilization of the tip of the cinch tube to avoid crumpling it in an undesired/unintended way.

Alternatively, the centering device of the cinch tube may be activated during retracting the tether with the implant into proximal direction such that the proximal end of the implant acting on the distal end of the cinch tube thereby folding the distal section of the cinch tube forming at least one knee that protrudes outwards from the cinch tube. It is explained above that the slitted distal end of the cinch tube is folded when the tethered implant abuts the distal end of the cinch tube.

In one embodiment the centering device may be deactivated, which means that the outer diameter is reduced in order to remove the recaptured implant from the patient's body, by retracting the cinch tube into or within the outer sheath of the catheter assembly. Thereby, the centering device may return from its second state to its first state (e.g. by transferring the plurality of radial arms from the second state to the first state) or an element of the centering device (e.g. the ring) may return from its second position to its first position as described above.

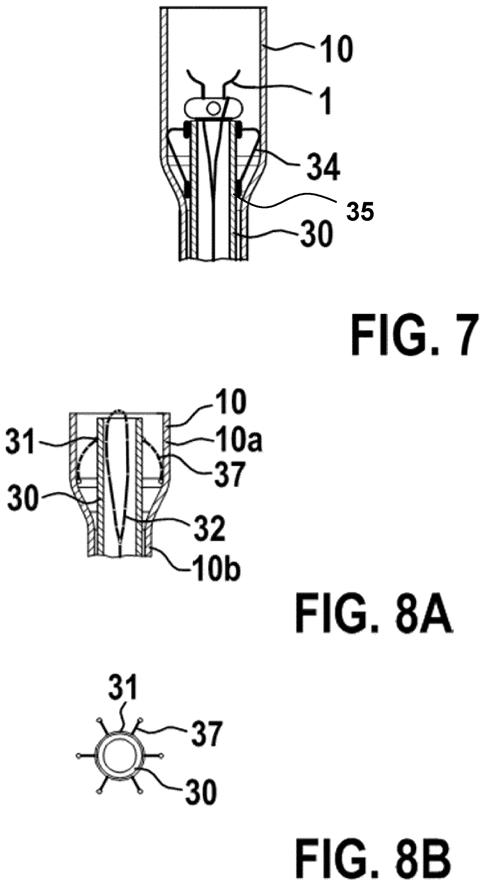

A full and enabling disclosure of the present invention, including the best mode thereof, directed to one of ordinary skill in the art is set forth in the following specification of the preferred embodiments. Thereby, further features and advantages are presented that are part of the present invention independently of the features mentioned in the dependent claims. The invention is defined in claim 1. The embodiments shown in figures 8, 9, 12, 13 are not part of the invention.

The specification refers to the accompanying Figures showing schematically
- Fig. 1: a longitudinal section of the distal end of an embodiment of a catheter system with an implantation assembly as a modular insert in a first step during implantation of a leadless cardiac pacemaker,
- Fig.2: the elements of Fig. 1 in a second step during implantation,
- Fig. 3: the elements of Fig. 1 in a third step during implantation,
- Fig. 4: a longitudinal section of the distal end of the catheter system of Fig. 1 with a first embodiment of a recapture assembly as modular insert in a first step during retrieval of a leadless cardiac pacemaker,
- Fig. 5a - 5e: the elements of Fig. 4 in further steps during retrieval,
- Fig. 6: the elements of Fig. 4 in a another step during retrieval,
- Fig. 7: the elements of Fig. 4 in yet a another step during retrieval,
- Fig. 8: the distal end of the catheter system of Fig. 1 with a second embodiment of a recapture assembly as a modular insert in a) a longitudinal section and b) a top view (recapture assembly only without snare), both in a first step during retrieval of a leadless cardiac pacemaker,
- Fig. 9: the elements of Fig. 8 in a) a longitudinal section and b) a top view (recapture assembly only without snare), both in a second step during retrieval of the leadless cardiac pacemaker,
- Fig. 10: the distal end of the catheter system of Fig. 1 with a third embodiment of a recapture assembly as a modular insert in a) a longitudinal section and b) a top view (recapture assembly only without snare) in the first step (analogous to Fig. 4) during retrieval of a leadless cardiac pacemaker,
- Fig. 11: the elements and view of Fig. 10 a) a longitudinal section and b) a top view (recapture assembly only without snare), both in a second step during retrieval of the leadless cardiac pacemaker,
- Fig. 12: a fourth embodiment of an recapture assembly with a leadless cardiac pacemaker in a side view, in a first step during retrieval of a leadless cardiac pacemaker, and
- Fig. 13: the elements of Fig. 12 in a second step during retrieval of the leadless cardiac pacemaker.

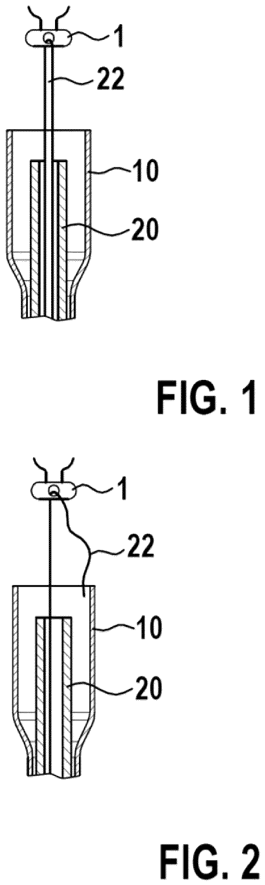

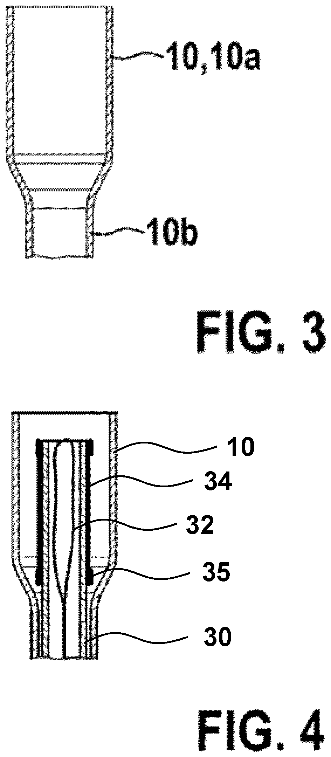

By using Fig. 1 to 3 an embodiment of a catheter system is described which does not only comprise a recapture assembly as indicated with regard to the other Fig. below but additionally comprises an implantation assembly. Fig. 1 to 3 depicts the catheter system during implantation of an implant, for example a leadless cardiac pacemaker 1 (the figures show only a proximal end of the leadless cardiac pacemaker 1). The catheter system comprises an outer sheath in form of a tube 10 which comprises at its distal end a section 10a with an enlarged inner diameter (see Fig. 3). Accordingly, the section 10b proximal to the section 10a has a smaller inner diameter. The outer tube 10 covers an implantation assembly as a modular insert of which one embodiment is shown in Fig. 1 to 3. The implantation assembly comprises a push tube 20 and a suture-like loop 22. The suture-like loop 22 is accommodated within the push tube 20, whereas the push tube 20 may mechanically translate/telescope relative to the outer sheath 10 of the catheter system. The suture-like loop 22 runs through a hitch of the cardiac pacemaker 1, wherein the hitch is provided at the proximal end of the cardiac pacemaker 1. The cardiac pacemaker 1 is transported with the catheter system to the implantation site, for example within a heart's ventricle. Subsequently after introducing the pacemaker 1 into the heart tissue the suture-like loop 22 releases the cardiac pacemaker 1 (see Fig. 1) after a cutter of the catheter system (not shown) had cut through one end of the suture-like loop 22 and removes the cardiac pacemaker 1 from the catheter system (see Fig. 2). After this, the push tube 20 and the suture-like loop 22 are removed from the outer sheath 10. This is shown in Fig. 3.

Accordingly, the push tube 20 is used to deploy the cardiac pacemaker 1 and facilitates anchoring engagement with the patient's myocardium. Following fixation of the cardiac pacemaker 1 within the heart, the push tube 20 can be retracted to allow the cardiac pacemaker 1 to maintain only a tethered connection to the catheter system by means of the compliant suture-like loop 22. The highly compliant suture-like loop 22 allows the cardiac pacemaker 1 to follow the free motion of the heart wall and to respond to hemodynamic forces without fully decoupling it from the implantation tooling. Decoupling the cardiac pacemaker 1 from the catheter system involves cutting the suture-like loop 22 and then unthreading it from the catheter system and cardiac pacemaker 1.

Recapture is then afforded by removing the push tube 20 and subsequently installing the recapture assembly within the catheter system as a modular insert which is shown in Fig. 4. The recapture assembly comprises a steerable cinch tube 30 with a tether in form of a snare 32 accommodated within. The cinch tube 30 according to a first embodiment shown in Fig. 4 to 7 comprises a plurality of wires 34, for example six wires 34, wherein a first, most distal end of each wire is attached to the distal end of the cinch tube 30 and the second, more proximal end of the wire 34 is attached to a slidable ring 35. The wires 34 are uniformly accommodated around the circumference of the cinch tube 30 and form a centering device. In the first position of the wires 34 shown in Fig. 4 at least a section of each wire 34 lies against the outer surface of the cinch tube 30.

To install the recapture assembly within the catheter system, the cinch tube 30 as well as the snare 32 are passed through the HCP-facing terminus of the catheter system distally towards the in-body cardiac pacemaker 1. During this process, the wires 34 reside in a non-deployed condition (or are compressed into a compliant condition capable of passing through the inner lumen of the baseline implantation tool) allowing ease of passage through the narrow inner lumen 10b of the outer tube 10.

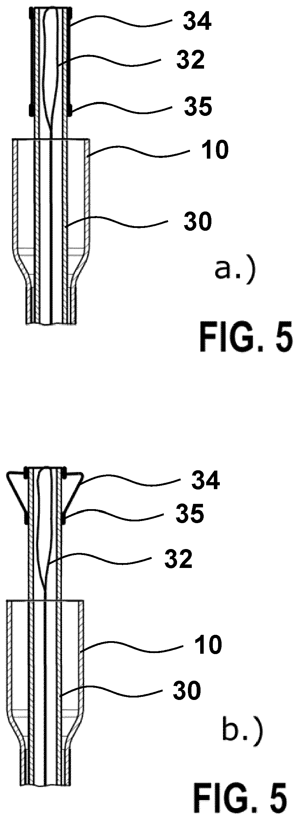

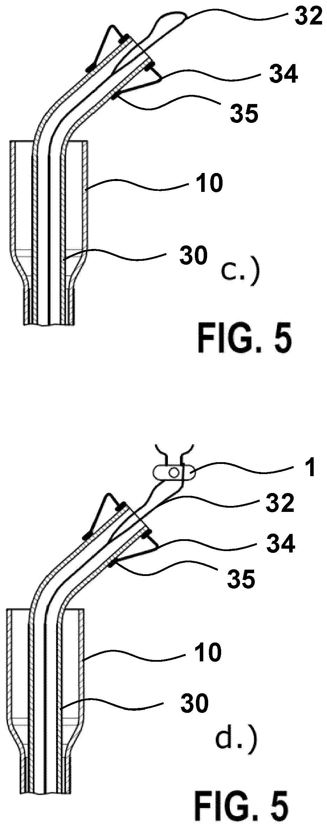

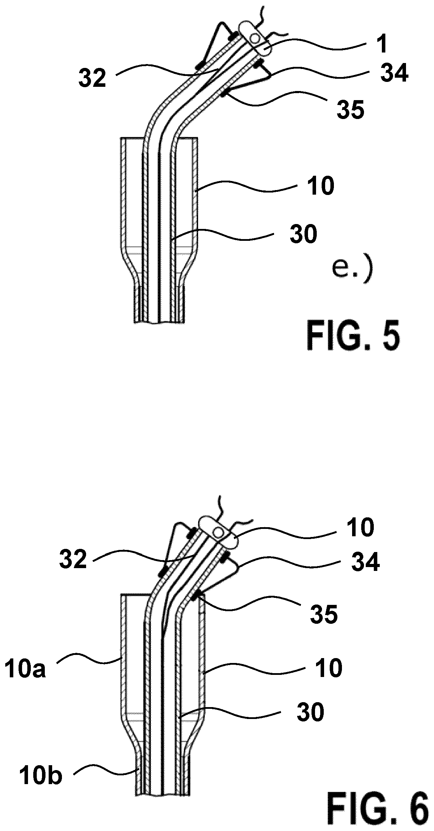

In the next step, which is shown in Fig. 5a - 5e and 6 the cinch tube 30 end the snare 32 are moved out of the distal end of the outer sheath 10 towards the cardiac pacemaker 1. In Fig. 5a, the cinch tube 30 is moved distally until it reaches out of the outer sheath 10. The wires 34 are expanded in order to form the centering device (Fig. 5b). The recapture snare 32 is moved out of the cinch tube 30 (Fig. 5c). In Fig. 5d, the recapture snare 32 reaches the implant and wraps around the T-shaped hitch of the cardiac pacemaker 1. The recapture snare 32 is pulled until the proximal end of the cardiac pacemaker 1 (e.g. the T-shaped hitch) is in direct contact with the distal end of the cinch tube 30 (Fig. 5e). The steerable, telescoping cinch tube 30 can be exercised to navigate to the implant in form of the cardiac pacemaker 1 and establish a tethered connection with the snare 32. Prior to, or in concert with, or following the tightening of the recapture snare 32 using the steerable, telescoping cinch tube 30 the distal terminus of the cinch tube 30 deploys its centering device (wires 34). For example, as shown in Fig. 6 the slidable ring 35 is shifted into distal direction relative to the cinch tube 30, for example by pulling a (not shown) wire connected to the slidable ring 35 or the cinch tube 30. Thereby each wire 34 is folded forming at least one knee that protrudes outwards from the cinch tube. By the centering device the recapture assembly with the cardiac pacemaker 1 is centered within the outer sheath 10 and the cardiac pacemaker 1 safely enters the outer sheath 10 and aligns with regard to the outer sheath 10 for back transport through the catheter (see Fig. 6 and 7).

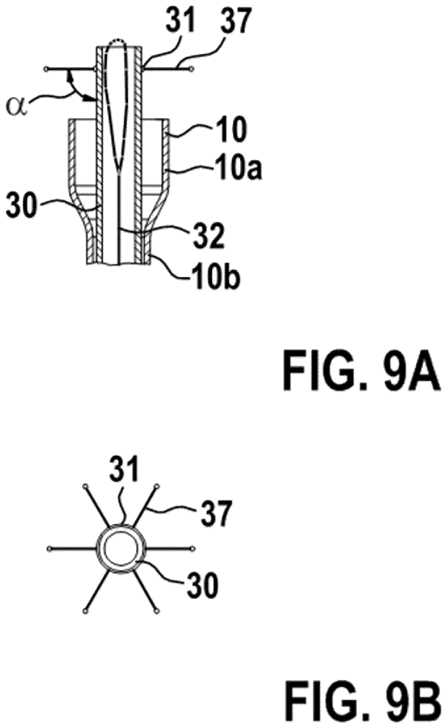

Fig. 8 and 9 show another embodiment of a recapture assembly with a different, less complex centering device. In this embodiment the centering device comprises a plurality of radial arms (spokes) 37 which are attached with its first end to the outer surface of the cinch tube 30 (for example by a ring or hub 31, in which the individual radial arms 37 fit). In a first position shown in Fig. 8 the radial arms 37 at least partially lie against the outer surface of the cinch tube 30 or run at least partly parallel to the outer surface of the cinch tube 30 and/or its longitudinal direction. In a second step, when the cinch tube 30 is moved into the enlarged section 10a of the outer tube 10 and out of the distal end of the outer tube 10 the biased radial arms 37 flex outwards in order to provide alignment of the cinch tube with the recaptured cardiac pacemaker because, for example, the radial arms 37 have a respective shape-memory configuration. The angle α between the radial arm 37 and a tangent to the outer surface of the cinch tube 30 at the attachment point of the radial arm 37 (see Fig. 9a)), wherein the tangent is parallel to the longitudinal axis of the cinch tube 30) may be between 60° and 150°, for example about 90°.

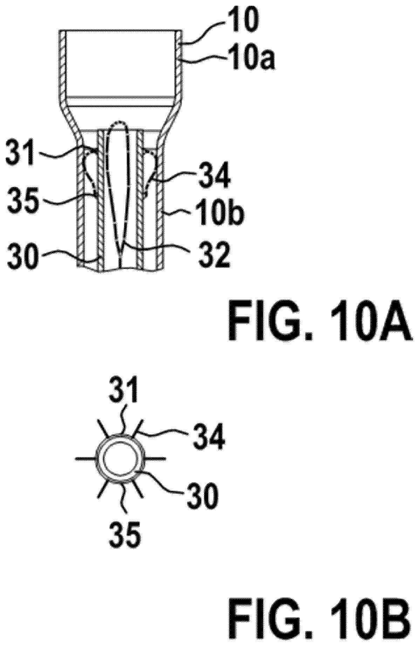

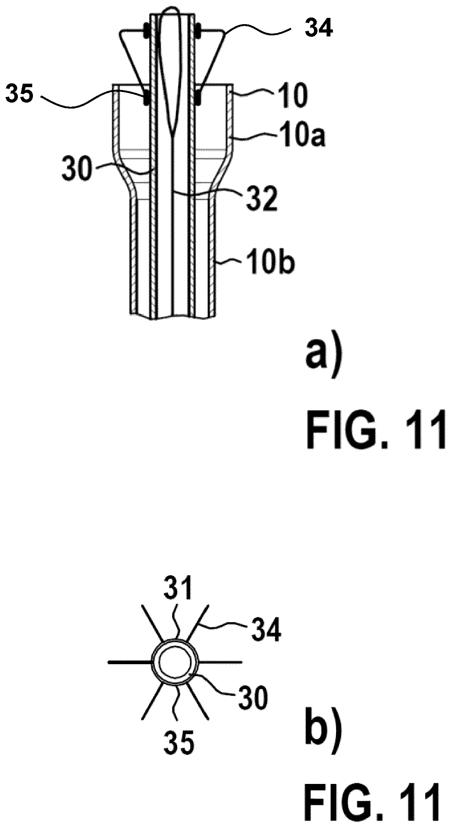

The third embodiment shown in Fig. 10 and 11 is similar to the embodiment shown in Fig. 4 to 7 with regard to the configuration of the cinch tube 30 and the wires 34. However, the activation of the centering device in this embodiment occurs when the cinch tube 30 is slid from a first position within the outer tube section with the smaller diameter 10b (see Fig. 10) with regard to the outer tube 10 such that the wires 34 are located within the enlarged distal section 10a of the outer tube 10 or distally from the distal end of the outer tube 10. The wires are biased or are provided with a shape-memory configuration such that they auto-deploy as soon as they reach the enlarged diameter section 10a of the outer tube 10 and/or pass the distal end of the outer tube 10. The deployment of the wires 34 is provided by offering a larger, non-compressing inner diameter by the outer sheath 10 (see Fig. 11). The recapture of the implant 1 may be performed similar to the first embodiment shown in Fig. 4 to 7.

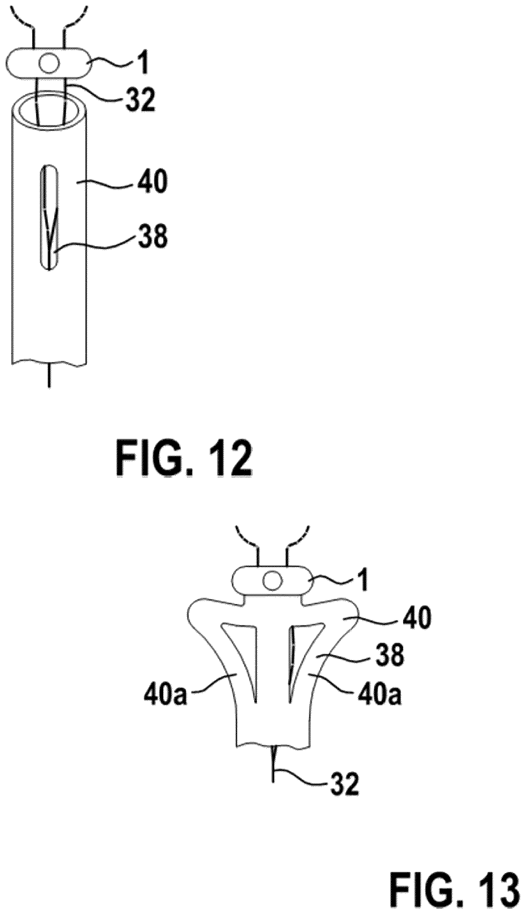

A fourth embodiment of a recapture assembly is shown in Fig. 12 and 13. In this embodiment a cinch tube 40 comprises a plurality of through-going slits 38 running parallel to the longitudinal axis of the cinch tube 40. The distal end of each slit 38 has a certain distance from the distal rim of the cinch tube 40. Fig. 12 shows a first position which is taken by the cinch tube 40 during installing the recapture assembly within the catheter system. During retrieval of the cardiac pacemaker 1, when the pacemaker 1 is pulled by the snare 32 toward the catheter system, the hitch-like end of the cardiac pacemaker abuts to and presses against the distal end of the cinch tube 40. By this force along the proximal direction the bands 40a separated by the slits 38 of the distal section of the cinch tube 40 are bent or folded forming at least one knee that radially protrudes outwards from the cinch tube 40 forming a centering device (see Fig. 13).

The catheter system, the recapture assembly and the method ease surgical implantation and extraction by shortening the otherwise necessary rigid in-catheter length by eliminating the presence of an alignment cup during implantation procedures, easing implantation procedures and avoiding unwanted additional pressure on device length reduction. The recapture assembly in its modular structure further avoids delivery tooling complexity and reduces intraoperative risk of user error by avoiding interaction with the steering functionalities native to the implantation tool (implantation assembly) and by presenting the retrieval controls only when retrieval is required. The system also reduces costs by separating retrieval assembly complexities from the implantation assembly.

### REFERENCE NUMBERS

- 1: leadless cardiac pacemaker
- 10: outer tube
- 10a: distal section of outer tube 10
- 10b: another section proximal to distal section 10a of outer tube 10
- 20: push tube
- 22: suture-like loop
- 30, 40: cinch tube
- 31: ring
- 32: snare
- 34: wire
- 35: slidable ring
- 37: radial arm
- 38: through-going slit
- 40a: band
- α: angle

## Claims

1. A recapture assembly for a catheter system comprising:
- a cinch tube (30) with a centering device provided at the distal end of the cinch tube (30); and
- a tether (32) located within the cinch tube (30) and adapted to be telescoped relative to the cinch tube (30);
**characterized in that**
the centering device comprises a plurality of wires (34), each wire (34) having a first end and a second end, wherein the first end of each wire (34) is fixedly attached to the distal end of the cinch tube (30) or a fixed ring accommodated at the outer surface of the cinch tube and the second end of each wire (34) is slidably arranged on the outer surface of the cinch tube (30), wherein the second end is slidably arranged on the cinch tube (30) between a first, most proximal position and a second, more distal position, and wherein in the first position each wire (34) deflects at least partially against the outer surface of the cinch tube (30) and in the second position each wire (34) deflects radially outwards and thereby forming a knee, that protrudes radially outwards from the cinch tube (30).

2. The recapture assembly according to claim 1, wherein the centering device is adapted to be activated by moving the cinch tube (30) and/or the tether (32) and/or a pulling element.

3. The recapture assembly according to claim 1 or 2, wherein the tether can be configured as snare.

4. The recapture assembly according to claim 1 to 3, wherein the second end of each wire (37) is attached to a ring (35) accommodated on the outer surface of the cinch tube (30), wherein the ring is slidable arranged on the cinch tube (30) between the first, most proximal position and the second, more distal position.

5. The recapture assembly according to any of the preceding claims, wherein the cinch tube (30) is configured to be steerable.

6. A catheter system comprising at least one outer sheath (10) and the recapture assembly of one of the preceding claims as a modular insert adapted to be passed through the outer sheath (10).

7. The catheter system of claim 6, wherein the recapture assembly is adapted to telescope from the distal end of the outer sheath (10).

8. The catheter system of any of claims 6 to 7, wherein an inner diameter of a distal section (10a) of the outer sheath (10) is greater than an inner diameter of another section (10b) accommodated proximally to the distal section of the outer sheath (10).

## Patentansprüche

1. Rückholbaugruppe für ein Kathetersystem, umfassend:
- ein Einfassrohr (30) mit einer Zentriervorrichtung, die an dem distalen Ende des Einfassrohrs (30) angeordnet ist; und
- eine Leine (32), die innerhalb des Einfassrohrs (30) angeordnet ist und eingerichtet ist, in Bezug auf das Einfassrohr (30) teleskopisch ausgefahren zu werden,
**dadurch gekennzeichnet, dass**
die Zentriervorrichtung eine Mehrzahl von Drähten (34) umfasst, wobei jeder Draht (34) ein erstes Ende und ein zweites Ende aufweist, wobei das erste Ende jedes Drahtes (34) fest an dem distalen Ende des Einfassrohrs (30) oder einem fixierten Ring, der an der Außenfläche des Einfassrohrs (30) angeordnet ist, angebracht ist und das zweite Ende jedes Drahtes (34) verschiebbar an der Außenfläche des Einfassrohrs (30) angeordnet ist, wobei das zweite Ende zwischen einer ersten, äußerst proximalen Position und einer zweiten, mehr distalen Position verschiebbar an dem Einfassrohr (30) angeordnet ist, und wobei in der ersten Position jeder Draht (34) zumindest zum Teil zu der Außenfläche des Einfassrohrs (30) hin abgelenkt ist und in der zweiten Position jeder Draht (34) radial auswärts abgelenkt ist und dadurch ein Knie formt, das von dem Einfassrohr (30) radial nach außen vorsteht.

2. Rückholbaugruppe nach Anspruch 1, wobei die Zentriervorrichtung eingerichtet ist, durch Bewegen des Einfassrohrs (30) und/oder der Leine (32) und/oder eines Ziehelements aktiviert zu werden.

3. Rückholbaugruppe nach Anspruch 1 oder 2, wobei die Leine als Schlinge konfiguriert sein kann.

4. Rückholbaugruppe nach Anspruch 1 bis 3, wobei das zweite Ende jedes Drahtes (37) an einem Ring (35) angebracht ist, der an der Außenfläche des Einfassrohrs (30) angeordnet ist, wobei der Ring zwischen der ersten, äußerst proximalen Position und der zweiten, mehr distalen Position verschiebbar an dem Einfassrohr (30) angeordnet ist.

5. Rückholbaugruppe nach einem der vorangehenden Ansprüche, wobei das Einfassrohr (30) eingerichtet ist, lenkbar zu sein.

6. Kathetersystem, mindestens eine äußere Hülse (10) und die Rückholbaugruppe nach einem der vorangehenden Ansprüche als modularen Einsatz umfassend, der eingerichtet ist, durch die äußere Hülse (10) geführt zu werden.

7. Kathetersystem nach Anspruch 6, wobei die Rückholbaugruppe eingerichtet ist zum teleskopischen Ausfahren aus dem distalen Ende der äußeren Hülse (10).

8. Kathetersystem nach einem der Ansprüche 6 bis 7, wobei ein Innendurchmesser eines distalen Abschnitts (10a) der äußeren Hülse (10) größer ist als ein Innendurchmesser eines anderen Abschnitts (10b), der proximal zu dem distalen Abschnitt der äußeren Hülle (10) angeordnet ist.

## Revendications

1. Ensemble de recapture pour un système de cathéter comprenant :
- un tube Cinch (30) avec un dispositif de centrage pourvu à l'extrémité distale du tube Cinch (30) ; et
- une attache (32) située à l'intérieur du tube Cinch (30) et adaptée pour être déployée de manière télescopique par rapport au tube Cinch (30) ;
**caractérisé en ce que**
le dispositif de centrage comprend une pluralité de fils (34), chaque fil (34) ayant une première extrémité et une seconde extrémité, dans lequel la première extrémité de chaque fil (34) est attachée fixement à l'extrémité distale du tube Cinch (30) ou à une bague fixe logée au niveau de la surface externe du tube Cinch et la seconde extrémité de chaque fil (34) est disposée de manière coulissante sur la surface externe du tube Cinch (30), dans lequel la seconde extrémité est disposée de manière coulissante sur le tube Cinch (30) entre une première position la plus proximale et une seconde position plus distale et dans lequel dans la première position chaque fil (34) est dévié au moins partiellement contre la surface externe du tube Cinch (30) et dans la seconde position chaque fil (34) est dévié de manière radiale vers l'extérieur formant ainsi un coude qui fait saillie de manière radiale vers l'extérieur depuis le tube Cinch (30).

2. Ensemble de recapture selon la revendication 1, dans lequel le dispositif de centrage est adapté pour être activé par déplacement du tube Cinch (30) et/ou de l'attache (32) et/ou d'un élément de tirage.

3. Ensemble de recapture selon la revendication 1 ou 2, dans lequel l'attache peut être conçue sous forme de collet.

4. Ensemble de recapture selon la revendication 1 à 3, dans lequel la seconde extrémité de chaque fil (37) est attachée à une bague (35) logée sur la surface externe du tube Cinch (30), dans lequel la bague est disposée de manière coulissante sur le tube Cinch (30) entre la première position la plus proximale et la seconde position plus distale.

5. Ensemble de recapture selon l'une quelconque des revendications précédentes, dans lequel le tube Cinch (30) est conçu pour être dirigeable.

6. Système de cathéter comprenant au moins une gaine externe (10) et l'ensemble de recapture selon l'une des revendications précédentes en tant qu'insert modulaire adapté à passer à travers la gaine externe (10).

7. Système de cathéter selon la revendication 6, dans lequel l'ensemble de recapture est adapté à se déployer de manière télescopique depuis l'extrémité distale de la gaine externe (10).

8. Système de cathéter selon l'une quelconque des revendications 6 à 7, dans lequel un diamètre interne d'une section distale (10a) de la gaine externe (10) est supérieur à un diamètre interne d'une autre section (10b) logée de manière proximale par rapport à la section distale de la gaine externe (10).
